# EUROPEAN PATENT SPECIFICATION

(11) **EP 1 557 185 B1**
(45) Date of publication and mention of the grant of the patent: **14.11.2007**
(21) Application number: 05000805.1
(22) Date of filing: 17.01.2005
(51) Int. Cl.: A61M 1/16, B01D 63/02, B01D 63/04

(54) **Device for treating blood for extracorporeal circulation**
Blutbehandlungsgerät für einen extrakorporellen Kreislauf
Dispositif de traitement de sang dans un circuit extracorporel

(30) Priority: 20.01.2004 IT MO20040012
(43) Date of publication of application: 27.07.2005
(73) Proprietor: Rand S.r.l., 41036 Medolla (Modena) (IT)
(72) Inventor: Galavotti, Daniele, 41037 Mirandola (Prov. of Modena) (IT)
(74) Representative: Feltrinelli, Secondo Andrea

(56) References cited:
- US-A- 4 639 353
- US-A- 5 706 889
- US-A- 5 817 279

## Description

The present invention relates to a device for treating blood for extracorporeal circulation.

The technique of cardiopulmonary bypass (assisted circulation or extracorporeal circulation) is currently known and allows temporary replacement of the gas exchange functions in lungs and of the pumping functions of the heart for treating severe cardiorespiratory failures and for performing heart surgery, in which the heart and/or the lungs must be available to the surgeon completely drained of blood.

This method substantially consists in drawing venous blood from the patient by means of at least one vena cava, collecting the blood inside a device that acts as a volumetric reservoir, sending it by means of a pump to an oxygenation device (or artificial lung), into which an appropriate gas mixture containing oxygen is injected, and in then sending it into the arterial system of the patient through the aorta.

If the operation is performed under hypothermia, so as to reduce the oxygen consumption of the main organs, the blood is treated in a heat exchanger before being returned to the patient.

In order to eliminate any bubbles formed due to less than optimum use of known devices, the blood can be made to pass through a settling chamber, also known as an antiembolism arterial filter, which is arranged downstream of the oxygenation device.

The oxygenation process that normally occurs inside the lungs, in which the air contained in the alveoli diffuses the oxygen to the blood that flows in the pulmonary capillaries, and in turn transfers carbon dioxide to the alveolar air, is reproduced inside the oxygenation device.

Different types of oxygenation devices are currently known.

So-called membrane oxygenation devices are known in particular which are substantially constituted by a box-like body for containing a chamber, which is provided with an intake port for the blood to be oxygenated (venous blood), with a discharge port for the oxygenated blood (arterial blood), with an intake passage for an oxygen-rich gas mixture and with a discharge passage for the carbon dioxide-rich gas mixture, and by a membrane, which is accommodated inside the chamber so that it is connected to the passages and is interposed between the ports, and is constituted by a bundle of hollow fibers inside which an oxygen-rich gas mixture flows.

The hollow fibers are completely immersed in the blood to be treated, which wets them externally, flowing in countercurrent or transversely to the gas mixture.

A wound heat exchanger oxygenator is also known in US 5,706,889 according to which a low prime, high heat exchange efficienty oxygenator is provided which includes an oxygenation portion and a heat exchange portion. Furthermore, an apparatus for processing fluids, in particular blood, is also known from US 5, 817, 279 which comprises a housing having a fluid inlet port and a fluid outlet port, with the housing being formed by an outher tube, an intermediate tube set whithin the outer tube, for forming a chamber therebetween, and an inner tube set whithin the intermediate for forming a second chamber therebetween.

It is also known that the materials used to manufacture the membranes must be hemocompatible (so as to avoid blood coagulation, plasma protein denaturation, and hemolysis), must ensure adequate gas transport, and must have mechanical strength characteristics that allow the manufacture and assembly of oxygenation devices and avoid any breakage thereof.

These membranes can be made, for example, of materials that have a microporous structure and are hydrophobic and permeable to blood gases (oxygen and carbon dioxide).

One known solution consists of an oxygenation device that has a cylindrical structure in which there are two cylindrical and coaxial walls, an internal wall and an external wall, which are suitable to delimit a chamber for accommodating a membrane constituted by at least one layer of hollow fibers arranged substantially longitudinally and in contact with the walls along their entire extension.

The hollow fibers are wet externally by the blood, which flows through the chamber in a substantially transverse direction, and are embedded, at their mutually opposite ends, in respective rings of polyurethane resin, known as "potting", one of which opens onto a chamber for the inflow of a gas mixture, the other end being connected to a chamber for the outflow of the mixture.

The gas mixture in input is rich in oxygen, which, in flowing along the capillaries is transferred to the blood and exchanged with carbon dioxide, which is carried towards the output.

However, these oxygenation devices are not free from drawbacks, including the fact that the flow of blood encounters considerable resistance in passing through the chamber that accommodates the membrane, causing significant load losses in the flow and a substantial mechanical stress on red cell membranes, which can cause their breakage with consequent hemolysis.

Moreover, oxygenation devices are known in which the membrane is constituted by a plurality of layers of hollow fibers wound around a framework provided with through openings for the passage of the blood to be oxygenated, so as to form a substantially tubular membrane.

Although this type of oxygenation device reduces the resistance encountered by the blood, it has the drawback that the lateral portions of the cross-section of the membrane that are arranged at the longitudinal comers of the framework are not crossed by the flow of blood, penalizing the exchange capacity of the device, with the consequent need to increase its dimensions and therefore the volume of blood removed from the patient during treatment.

Several studies are currently in progress to identify solutions that allow to optimize the performance of membrane oxygenation devices, increasing the transfer of oxygen to the blood for a given blood flow value, limiting the so-called priming volume of the device and hemolysis.

Moreover, heat exchange devices are known which also use capillary tubes.

In an exemplifying embodiment, the heat exchanger is in fact constructively similar to the oxygenation devices described above, with the difference that the hollow fibers that are used are made of a material that is impermeable to fluids (liquids and gases) and water is made to flow inside them to control the temperature of the blood of the patient.

These exchangers have the same drawbacks described above that arise from the limited heat exchange efficiency.

It should be noted that in currently known heart-lung machines, the heat exchanger (if provided) and the oxygenation device are usually constituted by respective devices arranged in series along the extracorporeal circulation line.

Devices for treating blood along extracorporeal circulation lines are also known which incorporate the heat exchanger and the oxygenation device.

In a known embodiment, this device is substantially constituted by a box-like body provided with a first chamber and a second chamber, which are mutually connected and respectively accommodate a first flat heat exchange membrane and a second flat oxygenation membrane; each chamber is provided with an intake port and with a discharge port for a fluid, which are associated with the corresponding membrane, the first port being provided with an intake passage for the blood to be treated and the second port being provided with a discharge passage for the oxygenated blood brought to the intended temperature.

However, even these combined devices are susceptible of further improvements.

The aim of the present invention is to eliminate the drawbacks noted above of known devices, by providing a blood treatment device for extracorporeal circulation that allows to promote and control in an optimum manner the exchange of gases between blood and gas mixture and/or the heat exchange between the blood and the temperature control fluid, avoiding the onset of hemolysis phenomena and optimizing the efficiency of such exchange.

Within this aim, an object of the present invention is to provide a device that minimizes the volume of blood that has to be drawn from the patient in order to allow its filling and operation (priming volume).

Another object of the present invention is to provide a device whose structure allows it to be perfused uniformly by the blood flow, so as to prevent any venous branching or stagnation phenomena, which can facilitate blood coagulation.

Another object of the present invention is to provide a device that is compact, easy to handle, has limited dimensions and is economically convenient, taking into account the fact that it is necessarily of the single-use type.

A further object of the present invention is to provide a device that is simple, relatively easy to provide in practice, safe in use, effective in operation, and has a relatively low cost.

This aim and these and other objects that will become better apparent hereinafter are achieved by the present blood treatment device for extracorporeal circulation as defined in the claims.

Further characteristics and advantages of the present invention will become better apparent from the following detailed description of two preferred but not exclusive embodiments of a device for treating blood for extracorporeal circulation, illustrated by way of non-limiting example in the accompanying drawings, wherein:
Figure 1 is a schematic sectional view, taken along a longitudinal plane, of a first embodiment of the device according to the invention;
Figure 2 is an enlarged-scale transverse sectional view, taken along the line II-II of Figure 1;
Figure 3 is a schematic sectional view, taken along a longitudinal plane, of a second embodiment of the device;
Figure 4 is an enlarged-scale transverse sectional view, taken along the line IV-IV of Figure 3.

With reference to the figures, the reference numeral 1 generally designates a device for treating blood for extracorporeal circulation.

The device 1 comprises a box-like body 2, which is substantially cylindrical and is designed to contain at least one chamber 3, which is associated with an intake port 4 for the blood to be treated, with a discharge port 5 for the blood treated inside said chamber, and with an intake passage 6 and a discharge passage 7 for a blood treatment fluid.

The box-like body 2 comprises a substantially tubular enclosure 8, which is closed at its opposite ends by respective caps 9 and 10, which are respectively associated, in the figures, with the upper and lower ends of the enclosure.

The ports 4 and 5 are offset on the side wall of the chamber 3 and lie on mutually opposite parts of the side wall.

Each one of the openings 6 and 7 is formed at one of the two caps 9 and 10.

The device 1 further comprises at least one substantially tubular membrane 11, which comprises a layer of hollow fibers that is closed in a loop or more preferably a plurality of mutually superimposed layers of hollow fibers of the type conventionally used in the biomedical field and made of hemocompatible material.

The membrane 11 is accommodated inside the chamber 3 and is arranged between the ports 4 and 5 so that the respective mutually opposite ends of the membrane are each connected to one of the passages 6 and 7.

The membrane 11 has a preferably elliptical flattened transverse cross-section, so as to reduce the dimensions of the device 1 and the volume of blood drawn from the patient.

The hollow fibers that constitute the membrane 11 are preferably arranged substantially parallel to each other, but other arrangements, such as interweaving or staggered fibers, are not excluded.

The fluid flows inside the hollow fibers from the intake passage 6 toward the discharge passage 7 and crosses the chamber 3 in a substantially longitudinal direction.

The blood, optionally with the addition of anticoagulant, instead flows in a substantially transverse direction with respect to the membrane 11 and therefore to the chamber 3, wetting externally its hollow fibers from the intake port 4 toward the discharge port 5.

According to the invention, the device 1 comprises at least one pair of containment elements 12 and 13, conventionally known as "potting", which are accommodated hermetically inside the chamber 3 proximate to the mutually opposite ends of the membrane 11 and in which the ends of the hollow fibers are inserted so as to substantially pass through in order to maintain the substantially tubular configuration of said membrane.

In the figures, the containment elements 12 and 13 are associated hermetically at the upper and lower ends of the enclosure 8 respectively.

The containment elements 12 and 13 are preferably made of polyurethane resin or the like.

In this manner, the membrane 11 can maintain its configuration without having to provide continuous containment walls, so as to minimize the resistance encountered by the flow of blood to be treated.

A compartment for the inflow (or outflow) of the fluid used is formed between the cap 9 and the containment element 12; moreover, between the cap 10 and the containment element 13 there is a compartment for the outflow (or inflow) of the fluid.

The containment elements 12 and 13 allow to avoid leakage of blood toward such compartments and thus avoid contamination thereof.

According to the invention, moreover, the device 1 comprises at least one substantially plate-like baffle insert 14, which is accommodated inside the membrane 11 (where the term "inside" is used to designate the portion of space delimited by the internal wall of said membrane) so that the upper and lower mutually opposite ends are respectively associated with the containment elements 12 and 13, and is arranged substantially transversely to the flow of blood.

The baffle insert 14 is suitable to facilitate the diffusion of the blood over the entire transverse cross-section of the membrane 11, so as to optimize exchange efficiency and avoid the formation of blood stagnation regions.

The baffle insert 14 preferably has a transverse extension that arranges it in contact with the membrane 11 at least at the longitudinal end portions; the baffle insert 14 accordingly acts as a diaphragm, preventing the substantially rectilinear flow of the blood through the membrane 11.

Conveniently, the box-like body 2 is provided with a plurality of longitudinal ridges 15, which protrude inside the chamber 3 and are arranged at least partially in contact with the membrane 11; a free loop is formed between two consecutive ridges 15, and the blood can flow therein so as to encounter limited resistance.

The internal surface of the enclosure 8 is in fact associated with a plurality of contoured ridges 15a, which duplicate the external surface of the membrane 11.

Advantageously, depending on the type of the hollow fibers that constitute the membrane 11, the device 1 can be of the type of an oxygenation device or of a heat exchanger.

If the hollow fibers are made of conventional plastic material that is impermeable to liquids and gases and the fluid used is constituted by a blood temperature control medium, such as water kept at an appropriate temperature, the device 1 is of the type of a heat exchanger.

If instead the hollow fibers are made of a conventional material that is microporous, hydrophobic and permeable to blood gases (oxygen and carbon dioxide) and the fluid used is constituted by an oxygen-rich gas mixture, the device 1 is of the type of an oxygenation device.

In this case, the device 1 may be provided with an additional passage 16 for discharge of the treated blood, which is formed in the enclosure 8 and can be associated with a conventional settling chamber to eliminate any bubbles.

In an advantageous embodiment, shown in the figures, the box-like body 2 forms two chambers 3, which are arranged in series and are mutually connected: a first chamber 3a for regulating the temperature of the blood to be treated and a second chamber 3b for oxygenating the blood.

The first and second chambers 3a and 3b, respectively, are provided with respective intake ports 4a and 4b, with respective discharge ports 5a and 5b, with respective intake passages 6a and 6b, and with respective discharge passages 7a and 7b.

The box-like body 2 is provided with a dividing wall 17, which is accommodated inside the enclosure 8 so as to separate the two chambers 3 and in which there is a through opening 18 that connects the chambers.

The enclosure 8 and the dividing wall 17 are preferably formed monolithically.

At the dividing wall 17, the caps 9 and 10 are provided with respective tabs 9a and 10a, which protrude into the containment elements 12 and 13 respectively and avoid contaminations of the fluids used in the two chambers 3.

The opening 18 coincides with the discharge port 5a and with the intake port 4b and is formed in the upper portion of the dividing wall 17.

The intake port 4a and the discharge port 5b are formed in the lower portion of the enclosure 8, so that the blood traces a substantially rising path along the first chamber 3a and then a substantially descending path along the second chamber 3b.

The openings 6a and 7a are formed respectively in the caps 10 and 9 and the temperature control medium flows upward along the first chamber 3a with reference to the figures.

The openings 6b and 7b are formed respectively in the caps 9 and 10, the gas mixture flowing downward along the second chamber 3b with reference to the figures.

The device 1 further comprises two membranes 11: a first membrane 11a accommodated in the first chamber 3a and a second membrane 11b accommodated in the second chamber 3b.

The first membrane 11 a and the second membrane 11b are provided by means of hollow fibers, respectively made of a material that is impermeable to liquids and gases and of a material of the type that is microporous, hydrophobic and permeable to blood gases.

A baffle insert 14 is accommodated inside each membrane 11; the baffle inserts 14 used in the two chambers are mutually substantially identical.

In this case, the device 1 is of the combined type: the first section comprising the first chamber 3a acts as a heat exchanger and the second section comprises the second chamber 3b acting as an oxygenation device.

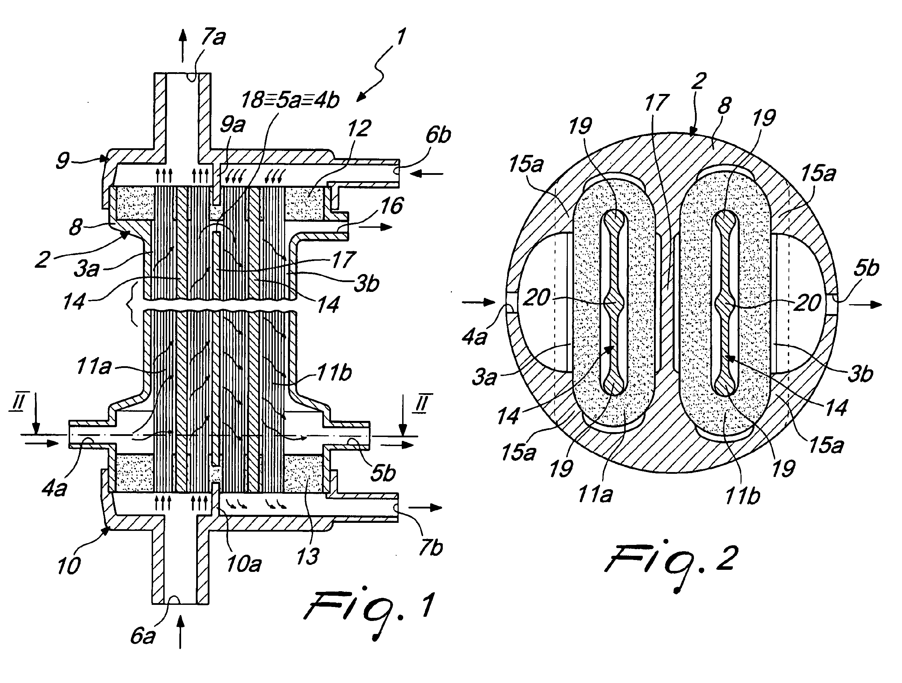

In a first embodiment, shown in Figures 1 and 2, each baffle insert 14 has longitudinal end portions that are contoured so as to form respective rounded beads 19, which are arranged substantially tangent inside the corresponding membrane 11.

Further, each baffle insert 14 has at least one longitudinal rib 20 that protrudes from at least one of its faces and imparts an undulating motion to the stream of blood comprised between the corresponding membrane 11 and said insert.

Each rib 20 can optionally be tangent to the corresponding membrane 11.

Each baffle insert 14 shown has a rounded contoured rib 20, which is arranged at its centerline and protrudes from both of its faces.

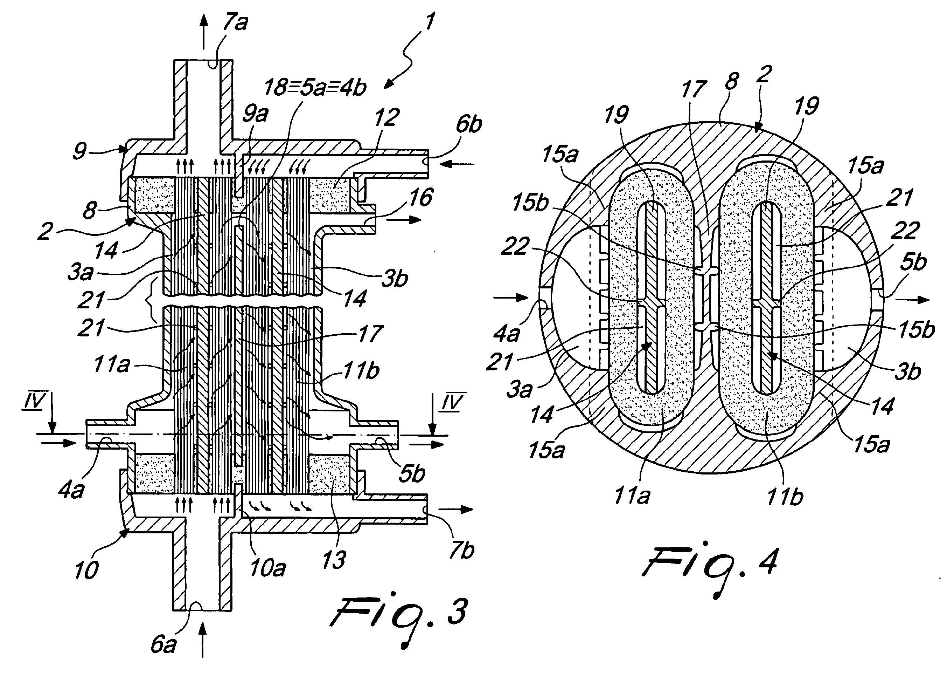

In a second embodiment (not illustrating the invention as defined in the claims), shown in Figures 3 and 4, each baffle insert 14 is associated with a plurality of relief profiles 21, which are distributed on it substantially at right angles to the insert.

Preferably, the profiles 21 are constituted by perimetric protrusions, which are distributed along the length of each baffle insert 14 with a substantially constant pitch and are at least partially in contact with the internal surface of the corresponding membrane 11.

In the figures, the profiles 21 are shown in contact along their entire perimeter with the internal surfaces of the membranes 11, but alternative embodiments are also possible in which the profiles are only partially tangent to the surfaces.

Each baffle insert 14, in this case also, is provided with a longitudinal rib 22 that protrudes from both of its faces until it touches the internal wall of the corresponding membrane.

In an alternative embodiment, the transverse extension of the ribs 22 may be smaller, so that they remain spaced from the membranes 11.

In this case, the dividing wall 17 is provided with ridges 15b on both sides, such ridges being rounded and tangent externally with respect to the membranes 11.

In the figures, the path traced by the blood is shown by means of arrows.

The heat exchange and/or oxygenation mechanism used in the device 1 is fully conventional and therefore the corresponding operation is assumed to be straightforwardly understandable for the person skilled in the art.

The diameter of the hollow fibers used, their density and the arrangement and the overall dimensions of the device 1 may vary according to the type of use to which it is dedicated.

Moreover, it is noted that the industrial manufacture of the device according to the invention is particularly easy and economically convenient.

In practice it has been found that the described invention achieves the intended aim and objects.

In particular, it is noted that the device according to the invention allows to provide a blood circulation which, by virtue of the presence of large passages, has limited load losses and allows to work at low pressures, ensuring quite negligible hemolytic damage.

Moreover, according to the invention, it is possible to obtain a combined device that is compact, efficient and of practical use.

The invention thus conceived is susceptible of numerous modifications and variations, all of which are within the scope of the appended claims.

In practice, the materials used, as well as the shapes and dimensions, may be any according to requirements without thereby abandoning the scope of the protection of the appended claims.

Where technical features mentioned in any claim are followed by reference signs, those reference signs have been included for the sole purpose of increasing the intelligibility of the claims and accordingly, such reference signs do not have any limiting effect on the interpretation of each element identified by way of example by such reference signs.

## Claims

1. - A blood treatment device for extracorporeal circulation, comprising a box-like body (2) for containing at least one chamber (3), which is associated with an intake port (4) for the blood to be treated, with a discharge port (5) for the treated blood, and with an intake passage (6) and a discharge passage (7) for a fluid, and at least one substantially tubular membrane (11), which comprises at least one layer of hollow fibres, is accommodated inside said chamber (3) and is arranged between said ports (4, 5) so that mutually opposite ends of the membrane (11) are each connected to one of said passages (6, 7), the fluid flowing inside said hollow fibres from said intake passage (6) toward said discharge passage (7) and the blood flowing substantially transversely to said hollow fibres and externally thereto from said intake port (4) toward said discharge port (5); at least one pair of containment elements (12, 13) which are accommodated hermetically within said chamber (3) proximate to the mutually opposite ends of said membrane (11), and at least one substantially plate-like baffle insert (14) which is accommodated inside said membrane (11) so that its mutually opposite ends are each associated with a respective containment element (12, 13) and is arranged substantially transversely to he flow of he blood, **characterized in that** said baffle insert (14) has a solid body provided with longitudinal end portions that are shaped so as to form respective rounded beads (19) which are arranged substantially tangent inside said membrane (11), said solid body being suitable to facilitate the diffusion of the blood over the entire transverse cross-section of said membrane (11).

2. The device according to claim 1, **characterized in that** said baffle insert (14) is in contact at least at the longitudinal end portions with said membrane (11).

3. The device according to claim 1 or 2, **characterized in that** said membrane (11) has a substantially elliptical transverse cross-section.

4. The device according to claim 1, **characterized in that** said baffle insert (14) comprises at least one substantially longitudinal rib (20) that protrudes from at least one of its faces.

5. The device according to claim 1, **characterized in that** it comprises a plurality of profiles (21) in relief that are distributed on said baffle insert (14).

6. The device according to claim 5, **characterized in that** said profiles (21) are arranged substantially transversely with respect to said baffle insert (14).

7. The device according to claim 5, **characterized in that** said profiles (21) are constituted by perimetric protrusions distributed along the length of said baffle insert (14).

8. The device according to claim 5, **characterized in that** said profiles (21) are at least partially tangent internally with respect to said membrane (11).

9. The device according to claim 5, **characterized in that** said profiles (21) are at least partially in contact with the internal surface of said membrane (11).

10. The device according to claim 1, **characterized in that** said hollow fibers are made of a plastic material that is impermeable to liquids and gases and said fluid is a means for blood temperature control.

11. The device according to claim 1, **characterized in that** said hollow fibers are made of microporous, hydrophobic and gas-permeable material and said fluid is a gas mixture that comprises oxygen.

12. The device according to claim 1, **characterized in that** said membrane (11) comprises a plurality of said layers, arranged so that they are mutually superimposed.

13. The device according to claim 1, **characterized in that** said hollow fibers are mutually substantially parallel, staggered or interwoven.

14. The device according to claim 1, **characterized in that** it comprises a plurality of substantially longitudinal ridges (15), which are associated with said box-like body (2), protrude into said chamber (3) and are arranged at least partially in contact with the outer surface of said membrane (11).

15. The device according to claim 1, **characterized in that** said longitudinal ridges (15) are contoured so as to substantially duplicate the outer surface of said membrane (11).

16. The device according to claims 14, **characterized in that** said longitudinal ridges (15) are contoured so as to be substantially tangent externally with respect to said membrane (11).

17. The device according to one or more of the preceding claims, **characterized in that** it comprises an additional blood outflow opening (16) which is associated with said chamber (3) and can be associated with a settling chamber for eliminating any bubbles.

18. The device according to claim 1, **characterized in that** said containment elements (12, 13) are made of polyurethane resin or the like.

19. The device according to claim 1, **characterized in that** said box-like body (2) comprises a substantially cylindrical enclosure (8) that is closed at its mutually opposite ends by respective caps (9,10).

20. The device according to claim 1, **characterized in that** said ports (4,5) associated with said enclosure (8) and are mutually staggered and arranged on opposite sides of said chamber (3), and **in that** said passages (6,7) are each associated with one of said caps (9, 10).

21. The device according to claim 1, **characterized in that** said box-like body (2) comprises two chambers (3), a first chamber (3a) and a second chamber (3b), which are arranged in series and are mutually connected, the discharge port (5) of the first chamber (3a) substantially mating with the intake port (4) of said second chamber (3b), and at least two of said membranes (11), a first and second membranes (11a, 11b), which are accommodated respectively within said first and second chambers (3a, 3b), said first and second membranes (11a,11b) being made respectively of a material of the type that is impermeable to liquids and gases and of the type that is microporous, hydrophobic and gas-permeable.

22. The device according to claim 21, **characterized in that** said box-like body (2) comprises a dividing wall (17) that is accommodated inside said enclosure (8) so as to form said two chambers (3a,3b) and is provided with a through opening (18) for the connection of said chambers (3a,3b).

23. The device according to claim 21, **characterized in that** the discharge port (5a) of said first chamber (3a), the discharge port (5b) of said second chamber (3b) and said through opening (18) substantially coincide.

24. The device according to claim 22 **characterized in that** said enclosure (8) and said dividing wall (17) are formed monolithically.

## Patentansprüche

1. Gerät zur Behandlung von Blut in einem Kreislauf außerhalb des Körpers mit einem kasten-ähnlichen Behälter (2), der mindestens eine Kammer (3) enthält, die mit einer Eingangsöffnung (4) für das zu behandelnde Blut versehen ist, mit einer Ausgangsöffnung (5) für das behandelte Blut und mit einem Eingangskanal (6) sowie einem Ausgangskanal (7) für ein Fluid und mit mindestens einer im Wesentlichen rohrförmigen Membran (11), die mindestens eine Lage von Hohlfasern umfasst, von der Kammer (3) aufgenommen und zwischen den Öffnungen (4, 5) angeordnet ist, derart, dass wechselseitig einander gegenüberliegende Endabschnitte der Membran (11) jeweils mit einem der Durchgangskanäle (6, 7) verbunden sind, wobei das Fluid innerhalb der Hohlfasern vom jeweiligen Eingangskanal (6) zu dem Ausgangskanal (7) strömt und das Blut im Wesentlichen quer zu den Hohlfasern und außerhalb der selben von dem genannten Eingangskanal (4) zu dem genannten Ausgangskanal (5) strömt; es ist mindestens ein Paar von Umfassungselementen (12, 13) vorgesehen, die hermetisch angepasst innerhalb der Kammer (3) in der Nähe der wechselseitig einander gegenüberliegenden Enden der Membran (11) angeordnet sind, sowie mindestens ein im Wesentlichen plattenförmiger Einsatz (4) zur Strömungslenkung, der innerhalb der Membran (11) so angebracht ist, dass seine einander gegenüber angeordneten Endabschnitte jeweils einem der Umfassungselemente (12, 13) zugeordnet sind und das Strömungsleitelement im Wesentlichen quer zur Strömungsrichtung des Blutes verlaufend angeordnet ist, **dadurch gekennzeichnet, dass** der zur Strömungsleitung dienende Einsatz (14) einen festen Grundkörper hat, der mit in Längsrichtung sich erstreckenden Endabschnitten versehen ist, die so gestaltet sind, dass die jeweils gerundete Würste (19) bilden, die im Wesentlichen derart angeordnet sind, dass sie die Membran (11) innenseitig berühren, wobei der feste Körper dazu dient, die Verteilung des Blutes über den gesamten transversalen Querschnitt der Membran (11) hinweg zu erleichtern.

2. Gerät nach Anspruch 1, **dadurch gekennzeichnet, dass** der Strömungsleiteinsatz (14) mit mindestens den sich in Längsrichtung sich erstreckenden Endabschnitten berührt die Membran (11) berührt.

3. Gerät nach Anspruch 1 und Anspruch 2, **dadurch gekennzeichnet, dass** die Membran (11) im Wesentlichen elliptischen transversalen Querschnitt hat.

4. Gerät nach Anspruch 1, **dadurch gekennzeichnet, dass** der Strömungsleiteinsatz (14) mindestens eine im Wesentlichen in Längsrichtung verlaufende Rippe (20) hat, die von mindestens einer seiner Begrenzungsflächen aufragt.

5. Gerät nach Anspruch 1, **dadurch gekennzeichnet, dass** es eine Mehrzahl von reliefartigen Profilierungen (21) umfasst, die verteilt auf dem Strömungsleiteinsatz (14) angeordnet sind.

6. Gerät nach Anspruch 5, **dadurch gekennzeichnet, dass** die Profilierungen (21) im Wesentlichen transversal bezüglich des Strömungsleiteinsatzes (14) angeordnet sind.

7. Gerät nach Anspruch 5, **dadurch gekennzeichnet, dass** die Profilierungen (21) durch am Umfang angeordnete Vorsprünge gebildet sind, die über die Länge des Strömungsleiteinsatzes (14) verteilt angeordnet sind.

8. Gerät nach Anspruch 5, **dadurch gekennzeichnet, dass** die Profilierungen mindestens teilweise die Membran (11) an deren Innenseite tangierend ausgebildet sind.

9. Gerät nach Anspruch 5, **dadurch gekennzeichnet, dass** die Profilierungen (21) mindestens teilweise in Berührungskontakt mit der inneren Begrenzungsfläche der Membran (11) stehen.

10. Gerät nach Anspruch 1, **dadurch gekennzeichnet, dass** die Hohlfasern aus einem Kunststoffmaterial bestehen, das für Flüssigkeiten und Gase nicht durchlässig ist und das Fluid ein Meduim für die Temperatur-Einstellung des Blutes ist.

11. Gerät nach Anspruch 1, **dadurch gekennzeichnet, dass** die Hohlfasern aus einem mikroporösen hydrophoben und gas-durchlässigen Material bestehen und das als Behandlungsfluid eine Gasmischung ist, die Sauerstoff enthält.

12. Gerät nach Anspruch 1, **dadurch gekennzeichnet, dass** die Membran (11) eine Mehrzahl von Hohlfaser-Lagen umfasst, die sich wechselseitig übereinander liegend angeordnet sind.

13. Gerät nach Anspruch 1, **dadurch gekennzeichnet, dass** die Hohlfasern wechselseitig zueinander im Wesentlichen parallel oder gegenseitig versetzt oder miteinander verwoben sind.

14. Gerät nach Anspruch 1, **dadurch gekennzeichnet, dass** es eine Mehrzahl von im Wesentlichen in Längsrichtung verlaufender Rippen (15) umfasst, die an dem kastenähnlichen Körper (2) angeordnet sind, in die Kammer (3) hineinragen und mindestens teilweise in Kontakt mit der äußeren Begrenzungsfläche der Membran (11) in Kontakt stehend angeordnet sind.

15. Gerät nach Anspruch 1, **dadurch gekennzeichnet, dass** die Längsrippen (15) derart berandet sind, dass sie die Außenfläche der Membran (11) im Wesentlichen verdoppeln.

16. Gerät nach Anspruch 14, **dadurch gekennzeichnet, dass** die Längsrippen (15) derartig konturiert sind, dass sie die Membran (11) im Wesentlichen an deren Außenseite berühren.

17. Gerät nach einem oder mehreren der vorhergehenden Ansprüche, **dadurch gekennzeichnet, dass** eine zusätzliche Blut-Ausflussöffnung (16) vorgesehen ist, die der Kammer (3) zugeordnet ist und mit einer Beruhigungskammer zur Eliminierung von Blasen verbindbar ist.

18. Gerät nach Anspruch 1, **dadurch gekennzeichnet, dass** die Umfassungselemente (12, 13) aus einem Polyurethanharz oder dergleichen gefertigt sind.

19. Gerät nach Anspruch 1, **dadurch gekennzeichnet, dass** der kastenförmige Grundkörper (2) ein im Wesentlichen zylindrisches Gehäuse (8) hat, das an seinen einander gegenüberliegenden Enden durch entsprechende Hauben (9, 10) verschlossen ist.

20. Gerät nach Anspruch 1, **dadurch gekennzeichnet, dass** die Ein- und Auslassöffnungen (4, 5) an dem Gehäuse (8) vorgesehen und gegeneinander versetzt an einander gegenüber liegenden Seiten der Kammer (3) angeordnet sind und dass die Durchlasskanäle (6, 7) jeweils je einer der Hauben (9, 10) zugeordnet sind.

21. Gerät nach Anspruch 1, **dadurch gekennzeichnet, dass** der kastenförmige Körper (2) zwei Kammern (3) umfasst, nämlich eine erste Kammer (3a) und eine zweite Kammer (3b), die aufeinander folgend angeordnet und miteinander verbunden sind, wobei die Auslassöffnung (5) der ersten Kammer (3a) im Wesentlichen mit der Einlassöffnung (4) und der zweiten Kammer (3b) gepaart ist, sowie mindestens zwei der genannten Membranen (11) umfasst, nämlich eine erste und eine zweite Membran (11a, 11 b), die jeweils innerhalb der ersten und der zweiten Kammer (3a, 3b) angeordnet sind, wobei die erste und die zweite Membran (11a, 11 b) jeweils aus einem Material bestehen, das für Flüssigkeiten und Gase undurchlässig ist beziehungsweise aus einem Material desjenigen Typs, das mirkoporös, hydrophob und as-durchlässig ist.

22. Gerät nach Anspruch 21, **dadurch gekennzeichnet, dass** der kastenförmige Körper (2) eine Trennwand (17) hat, die in das Gehäuse (8) derart eingesetzt ist, dass sie die beiden Kammern (3a, 3b) bildet und mit einer Durchgangsöffnung (18) zur Verbindung der beiden Kammern (3a, 3b) zu versehen ist.

23. Gerät nach Anspruch 21, **dadurch gekennzeichnet, dass** die Auslassöffnung (5a) der ersten Kammer (3a), die Auslassöffnung (5b) der zweiten Kammer (3b) und die Durchgangsöffnung (18) im Wesentlichen zusämmenfallen.

24. Gerät nach Anspruch 23, **dadurch gekennzeichnet, dass** das Gehäuse (8) und die Trennwand (17) einstückig ausgebildet sind.

## Revendications

1. Dispositif de traitement sanguin pour une circulation extracorporelle, comprenant un corps (2) en forme de boîtier destiné à contenir au moins une chambre (3), qui est associé à un orifice d'admission (4) pour le sang à traiter, avec un orifice de refoulement (5) pour le sang traité, et avec un passage d'admission (6) et un passage de refoulement (7) pour un fluide, et au moins une membrane sensiblement tubulaire (11), qui comprend au moins une couche de fibres creuses, est reçue à l'intérieur de ladite chambre (3) et est agencée entre lesdits orifices (4, 5) de telle sorte que les extrémités mutuellement opposées de la membrane (11) soient chacune reliées à l'un desdits passages (6, 7), le fluide circulant à l'intérieur desdites fibres creuses depuis ledit passage d'admission (6), en direction dudit passage de refoulement (7), et le sang s'écoulant sensiblement transversalement auxdites fibres creuses et extérieurement à celles-ci, depuis ledit orifice d'admission (4), en direction dudit orifice de refoulement (5) ; au moins une paire d'éléments de rétention (12, 13) qui sont reçus de manière hermétique à l'intérieur de ladite chambre (3) à proximité des extrémités mutuellement opposées de ladite membrane (11), et au moins un insert déflecteur (14) en forme de plaque qui est reçu à l'intérieur de ladite membrane (11) de telle façon que ses extrémités mutuellement opposées soient chacune associées à un élément de rétention (12, 13) et qui est placé sensiblement transversalement à la circulation du sang, ***caractérisé en ce que*** ledit insert déflecteur (14) possède un corps plein pourvu de portions terminales longitudinales qui sont conformées de manière à former des cordons arrondis respectifs (19) qui sont placés sensiblement tangentiellement à l'intérieur de ladite membrane (11), ledit corps solide étant apte à faciliter la diffusion du sang sur toute la section transversale de ladite membrane (11).

2. Dispositif selon la revendication 1, ***caractérisé en ce que*** ledit insert déflecteur (14) est en contact, au moins sur les portions terminales longitudinales, avec ladite membrane (11).

3. Dispositif selon la revendication 1 ou 2, ***caractérisé en ce que*** ladite membrane (11) possède une section transversale sensiblement elliptique.

4. Dispositif selon la revendication 1, ***caractérisé en ce que*** ledit insert déflecteur (14) comprend au moins une nervure sensiblement longitudinale (20) qui fait saillie sur au moins l'une de ses faces.

5. Dispositif selon la revendication 1, ***caractérisé en ce qu'**il* comprend une pluralité de profilés (21) en relief qui sont répartis sur ledit insert déflecteur (14).

6. Dispositif selon la revendication 5, ***caractérisé en ce que*** lesdits profilés (21) sont placés sensiblement transversalement audit insert déflecteur (14).

7. Dispositif selon la revendication 5, ***caractérisé en ce que*** lesdits profilés (21) sont constitués par des saillies périmétriques réparties sur la longueur de l'insert déflecteur (14).

8. Dispositif selon la revendication 5, ***caractérisé en ce que*** lesdits profilés (21) sont au moins partiellement tangents intérieurement à ladite membrane (11).

9. Dispositif selon la revendication 5, ***caractérisé en ce que*** lesdits profilés (21) sont au moins partiellement en contact avec la surface interne de ladite membrane (11).

10. Dispositif selon la revendication 1, ***caractérisé en ce que*** lesdites fibres creuses sont faites d'une matière plastique qui est imperméable aux liquides et aux gaz et ledit fluide est un moyen pour la régulation de la température sanguine.

11. Dispositif selon la revendication 1, ***caractérisé en ce que*** lesdites fibres creuses sont faites d'un matériau microporeux hydrophobe, perméable aux gaz, et ledit fluide est un mélange gazeux qui contient de l'oxygène.

12. Dispositif selon la revendication 1, ***caractérisé en ce que*** ladite membrane (11) comprend une pluralité desdites couches, disposées de telle sorte qu'elles soient superposées les unes aux autres.

13. Dispositif selon la revendication 1, ***caractérisé en ce que*** lesdites fibres creuses sont sensiblement parallèles, décalées ou entrecroisées les unes par rapport aux autres.

14. Dispositif selon la revendication 1, ***caractérisé en ce qu'**il* comprend une pluralité d'arêtes (15) sensiblement longitudinales qui sont associées audit corps (2) en forme de boîtier, font saillies dans ladite chambre (3) et sont disposées, au moins partiellement, en contact avec la surface extérieure de ladite membrane (11).

15. Dispositif selon la revendication 14, ***caractérisé en ce que*** lesdites arêtes longitudinales (15) sont profilées de manière à sensiblement dupliquer la surface extérieure de ladite membrane (11).

16. Dispositif selon la revendication 14, ***caractérisé en ce que*** lesdites arêtes longitudinales (15) sont profilées de manière à être sensiblement tangentes extérieurement à ladite membrane (11).

17. Dispositif selon l'une ou plusieurs des revendications précédentes, ***caractérisé en ce qu'**il* comprend une ouverture supplémentaire (16) d'écoulement de sortie du sang, qui est associée à ladite chambre (3) et peut être associée à une chambre de décantation pour éliminer toute bulle.

18. Dispositif selon la revendication 1, ***caractérisé en ce que*** lesdits éléments de rétention (12, 13) sont faits de résine polyuréthane ou similaire.

19. Dispositif selon la revendication 1, ***caractérisé en ce que*** ledit corps (2) en forme de boîtier comprend une enceinte sensiblement cylindrique (8) qui est fermée sur ses extrémités opposées par des couvercles respectifs (9, 10).

20. Dispositif selon la revendication 1, ***caractérisé en ce que*** lesdits orifices (4, 5) sont associés à ladite enceinte (8) et sont mutuellement décalés et situés sur des côtés opposés de ladite chambre (3), et ***en ce que*** lesdits passages (6, 7) sont chacun associés à l'un desdits couvercles (9, 10).

21. Dispositif selon la revendication 1, ***caractérisé en ce que*** ledit corps (2) en forme de boîtier comprend deux chambres (3), une première chambre (3a) et une deuxième chambre (3b) qui sont agencées en série et sont reliées entre elles, l'orifice de refoulement (5) de la première chambre (3a) s'accouplant sensiblement avec l'orifice d'entrée (4) de ladite deuxième chambre (3b) et au moins deux desdites membranes (11), une première et une deuxième membranes (11a, 11b) qui sont reçues respectivement à l'intérieur desdites première et deuxième chambres (3a, 3b), lesdites première et deuxième membranes (11a, 11b) étant faites respectivement d'un matériau du type imperméable aux liquides et aux gaz, et d'un matériau du type microporeux, hydrophobe et perméable aux gaz.

22. Dispositif selon la revendication 21, ***caractérisé en ce que*** ledit corps (2) en forme de boîtier comprend une cloison de séparation (17) qui est reçue à l'intérieur de ladite enceinte (8) de manière à former lesdites deux chambres (3a, 3b) et est munie d'une ouverture de passage (18) pour le raccordement desdites chambres (3a, 3b).

23. Dispositif selon la revendication 21, ***caractérisé en ce que*** l'orifice de refoulement (5a) de ladite première chambre (3a), l'orifice de refoulement (5b) de ladite deuxième chambre (3b) et ladite ouverture de passage (18) coïncident sensiblement.

24. Dispositif selon la revendication 22, ***caractérisé en ce que*** ladite enceinte (8) et ladite cloison de séparation (17) sont formées de manière monolithique.
